# EUROPEAN PATENT APPLICATION

(11) **EP 2 166 094 A1**
(43) Date of publication of application: **24.03.2010**
(21) Application number: 08305583.0
(22) Date of filing: 23.09.2008
(51) Int. Cl.: C12N 15/11, C07K 14/435, A61K 31/713

(54) **Methods for prolonging the health benefits triggered by a dietary restriction using a sphingosine kinase inhibitor**

(71) Applicant: ECOLE NORMALE SUPERIEURE DE LYON, 69364 Lyon Cedex 07 (FR); Centre National de la Recherche Scientifique, 75016 Paris Cedex 16 (FR); UNIVERSITE CLAUDE BERNARD - LYON 1, 69100 Villeurbanne (FR)
(72) Inventor: Aguilaniu, Hugo, 69006 Lyon (FR); Maitre, Marianne, 69003 Lyon (FR)
(74) Representative: Gallois, Valérie

(57) **Abstract**

The present invention relates to a method for prolonging the benefits of a Dietary Restriction by using a sphingosine kinase inhibitor and a method for identifying or screening a molecule suitable for prolonging the benefits of a Dietary Restriction.

SEQUENCE LISTING

<110>  Ecole Normale Supérieure de Lyon
       Centre National de la Recherche Scientifique
       Université Claude Bernard Lyon 1

<120>  Methods for prolonging the health benefits triggered by a Dietary
        Restriction using a sphingosine kinase inhibitor

<130>  B760EP

<160>  15

<170>  PatentIn version 3.3

<210>  1
<211>  21
<212>  DNA
<213>  artificial sequence

<220>
<223>  SPHK1 primer

<400>  1
acaggaacaa tgtcgtggaa a                                                21


<210>  2
<211>  21
<212>  DNA
<213>  artificial sequence

<220>
<223>  SPHK1 primer

<400>  2
tttatccaac ttcggtccaa c                                                21


<210>  3
<211>  20
<212>  DNA
<213>  artificial sequence

<220>
<223>  C34C6.4 primer

<400>  3
cggcacgatc tctacaaagg                                                  20


<210>  4
<211>  20
<212>  DNA
<213>  artificial sequence

<220>
<223>  C34C6.4 primer

<400>  4
agcacttcca gctccaacaa                                                  20


<210>  5
<211>  20
<212>  DNA
<213>  artificial sequence

<220>

<223>  DAF16 primer

<400>  5
tacgaatgga tggtccagaa                                                    20


<210>  6
<211>  20
<212>  DNA
<213>  artificial sequence

<220>
<223>  DAF16 primer

<400>  6
tcgcatgaaa cgagaatgaa                                                    20


<210>  7
<211>  20
<212>  DNA
<213>  artificial sequence

<220>
<223>  PHA4 primer

<400>  7
aacgtgagcc atcgagaaag                                                    20


<210>  8
<211>  20
<212>  DNA
<213>  artificial sequence

<220>
<223>  PHA4 primer

<400>  8
gatgatgtgg tcgtgatgga                                                    20


<210>  9
<211>  20
<212>  DNA
<213>  artificial sequence

<220>
<223>  AAK2 primer

<400>  9
agctcgtcgc ttctttcaac                                                    20


<210>  10
<211>  20
<212>  DNA
<213>  artificial sequence

<220>
<223>  AAK2 primer

<400>  10
caaagtccgc aatcttcaca                                                    20


<210>  11
<211>  20
<212>  DNA

<213>  artificial sequence

<220>
<223>  LET 363 primer

<400>  11
cgccctgatt ctcaatgaac                                                    20


<210>  12
<211>  20
<212>  DNA
<213>  artificial sequence

<220>
<223>  LET 363 primer

<400>  12
ggacaagcca ttcaacacct                                                    20


<210>  13
<211>  20
<212>  DNA
<213>  artificial sequence

<220>
<223>  ACT1  primer

<400>  13
cccactcaat ccaaaggcta                                                    20


<210>  14
<211>  20
<212>  DNA
<213>  artificial sequence

<220>
<223>  ACT1  primer

<400>  14
atctccagag tcgaggacga                                                    20


<210>  15
<211>  1125
<212>  DNA
<213>  artificial sequence

<220>
<223>  sequence encoding RNAi against sphk-1

<400>  15
aaagccgacc ttggaaagtt caatggggtt ttgattcttt ctggagatgg tttggttttc      60

gaggtaacgc tttcaaaaaa cacgaaatta gtttaaattc gttggaggca gtttttgaat     120

ttaaaaaatt atattattca cctttccgcc gaaataatgc aattttaatt tcaaatttgt     180

aaattttcag gctctcaacg gaattctttg tcgtgaagat gcattccgga tattcccaac     240

tctcccaatc ggaattgttc ccagcggatc cggaaacgga cttttatgtt cggttttgtc     300

gaaatacgga acaaaaatga acgagaaatc tgtaatggaa agagctttgg aaattgccac     360

ctcaccaaca gcaaaggcgg aaagcgtagc tttgtattca gttaaaacag ataatcagtc     420

atatgcatcg tttttgtcaa ttggatgggg tttgatggca gatattgaca ttgacagtga     480

gaaatggaga aaatcgcttg gccatcacag attcactgtt atgggattca ttcgcagctg 540
cagtaagatt ttaaaaaatt taaattattc taaaacttcc aattttcaga tctccgatct 600
tataaaggac gccttacgta tcgtccatac aaaccaaaag gattccatcc atcgtccaac 660
gttttcagtg tgtatgaaaa aactacacaa caaagggtga tcaatttcaa aaatatattt 720
tttaaaatag ttcttaacat atatttttta agtatttttt attagtaacg acccttccaa 780
taatttctca tttttctaga ttgatgattc aaaagtaaaa acgaatggat ccgtgtcaga 840
ttccgaagaa gaaacaatgg agacaaaatt ccaaaattgg acgctgcccg attcagatga 900
aactttggca gttggaagtt ctgatttaga agaaactgtc gtaatcgagg ataattttgt 960
gaatatctat gcagttactc tttcccatat cgcagctgat ggaccattcg ctccaagtgc 1020
caaattagaa gataatcgaa ttcatctttc gtatattcta tggaaagata tcggtacacg 1080
agttaatatt gctaaatatc ttttggcgat tgaacacgag actca 1125

## Description

### FIELD OF THE INVENTION

The present invention relates to the field of medicine, in particular to the treatment or prevention of ageing and obesity and to screening methods for molecules useful in this treatment.

### BACKGROUND OF THE INVENTION

Dietary Restriction (DR) consists in lowering daily food intake as much as possible without reaching malnutrition. This manipulation is beneficial since it lengthens lifespan and lowers the incidence of most age related diseases (cancer, neuro-degeneration, sarcopenia, etc...) and augments vitality. Interestingly, DR is effective in almost all species tested so far, suggesting that the "lifespan" response to lowered food intake is a conserved and thus, important adaptive trait. In addition, all observations related to DR that were initially made on model systems such as worms, flyes or even yeast have proven true in mammals.

In humans, simply applying DR seems difficult, given the severity of the required diet. Indeed, it was recently shown that harsh diets are psychologically challenging. Altogether, this makes it desirable to recapitulate the DR response without necessarily performing such a diet. That is why various attempts have been made (or are being made) to find drugs that could mimic the DR response.

Several attempts to recapitulate the DR response without performing a DR have been made.

Most notably, the molecule "Resveratrol" was initially thought to be a mimic of DR. However, it was recently shown to not be effective on animals that are normally fed, as initially hoped. Rather, it protects animals fed with a high fat diet against the adverse effects of over-feeding. Thus, as resveratrol clearly shows some advantages but it is no longer thought to be a chemical mimic of DR *stricto sensu.*

Another strategy is to affect appetite. Manipulation of the appetite hormone "Ghrelin" is also considered to be potentially useful pharmaceutically. Because injection of Ghrelin triggers appetite, scientists are now actively working on the development of Ghrelin antagonists that would acts oppositely and limit appetite. This would ultimately allow patients to eat less, thereby triggering a DR response. This strategy can be fruitful but must be heavily regulated as eating disorders are frequent and usually witness of psychological instability. We believe that affecting appetite will be of a limited use because of the risks of misuse that is inherent to this strategy.

Therefore, there is still a strong need to provide alternative anti-ageing treatments, and in particular a strategy to efficiently benefit from DR without its adverse side effects.

### SUMMARY OF THE INVENTION

The present invention provides means for maintaining individuals in a metabolic state that is beneficial for their health and that they acquired by performing a brief DR.

In particular, the present invention concerns an inhibitor of a sphingosine kinase for prolonging the benefits of a Dietary Restriction. It also concerns the use of an inhibitor of a sphingosine kinase for preparing a pharmaceutical or nutraceutical composition for prolonging the benefits of a Dietary Restriction. It further concerns a method for prolonging the benefits of a Dietary Restriction in a subject, comprising submitting the subject to a Dietary Restriction and administering to the subject an effective amount of an inhibitor of a sphingosine kinase during the Dietary Restriction and after the end of the Dietary Restriction. In particular, the benefits of a Dietary Restriction are selected from the group consisting of a decrease of the incidence of aging related pathologies and an increased vitality and leanness. Alternatively, the benefits can be a decrease of body mass. The present invention also concerns a product comprising a sphingosine kinase inhibitor and at least one component selected from the group consisting of beverage ingredients, food ingredients, animal feed ingredients, pet food ingredients, nutraceutical ingredients, dietary supplement ingredients, and functional food ingredients.

The present invention also concerns a method for identifying or screening a molecule suitable for prolonging the benefits of a Dietary Restriction, comprising: 1) providing adult worms with a normal fat content; 2) submitting the worms to food deprivation during about 4-7 hours in the presence of a test molecule; 3) cultivating the worms in an axenic medium containing the test molecule during about 15-25 hours; 4) determining the fat content of worms; and, 5) selecting the test compound if the fat content of worms is low, said selected compound being suitable for prolonging the benefits of a Dietary Restriction. Preferably, the worm is *C. elegans.* In particular, a worm with a normal fat content is dark and a worm with a low fat content is clear and the determining step 4) is based on this parameter. The method is preferably carried out in a multiple-well plate.

### BRIEF DESCRIPTION OF THE DRAWINGS

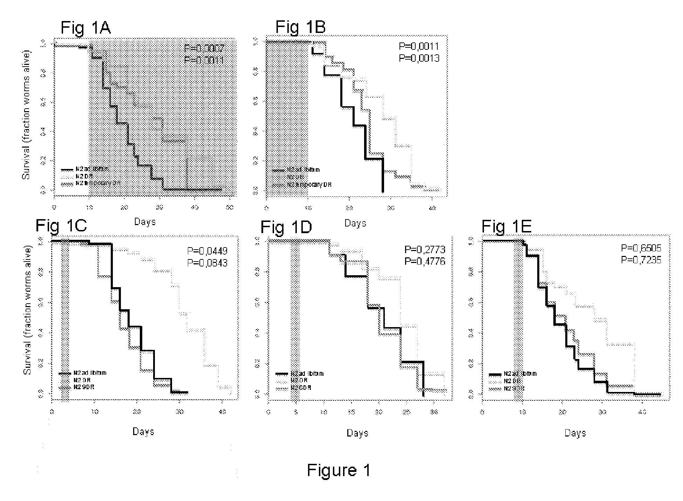

**Figure 1****:** Characterization of the temporal requirement of Dietary Restriction. Lifespan were performed as described previously on agar plates containing 5-fluorouracil (15µM) to prevent growth of the progeny. In all panels, black curves represent control conditions where worms were fed *ad libitum* during their whole life. In contrast, light grey curves depict worms that were submitted to DR, performed by the method of food deprivation from day 1 of adulthood until then end of the experiment. Curves in dark grey represent the survival curves of worms that were submitted to a temporary dietary restriction (food deprivation in our system). A shaded area on the graph represents the period of the diet. On panels A and B, temporary diets were long as indicated by the shaded area. On panels C, D and E, temporary diets are short dietary restriction (SDR) and represent a 2-days long DR. Probability indicated on the graph concern the comparison between the black curves and the dark grey curves analyzed with the Log Rank test on top and the Mendel cox test below.

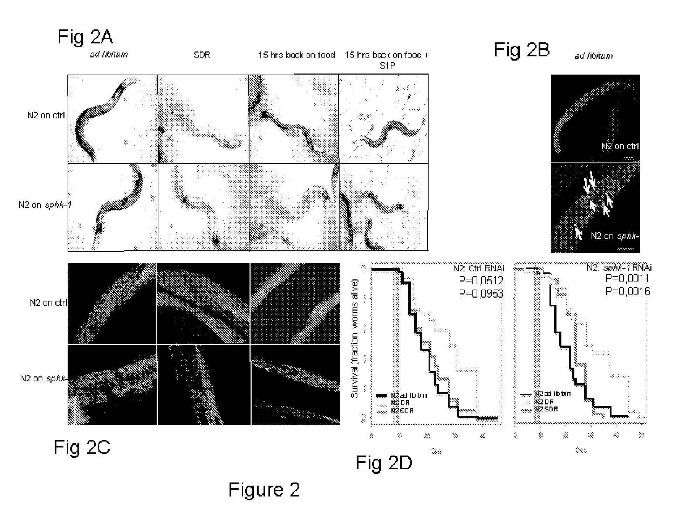

**Figure 2****:** Characterization of the effects of treatment by RNAi against *sphk-1.* Panel A shows the accumulation of fat by simple observation at the binocular loupe under conditions indicated in the panel. Panel B shows DAPI staining of isolated gonads on wild type worms fed either control RNAi (*ht115*) or *sphk-1* RNAi. White arrows indicate abnormally dense, crescent shaped nuclei, typical of mitotic division in a zone usually dedicated to meiosis. Panel C shows GFP labeled mitochondria of worms expressing the MYO-3::GFP construct under conditions indicated on the figure. Panel D shows lifespan analysis as described previously (Kenyon et al, 1993) on agar plates containing 5-fluorouracil (15µM) to prevent growth of the progeny. In all panels, black curves represent control conditions where worms were fed *ad libitum* during their whole life. In contrast, light grey curves depict worms that were submitted to DR, performed by the method of food deprivation from day 1 of adulthood until then end of the experiment. Curves in dark grey represent the survival curves of worms that were submitted to a short dietary restriction (SDR) food deprivation in our system). A shaded area on the graph represents the period of the diet. Strains and medium are specified on the graph. Probability indicated on the graph concern the comparison between the black curves and the dark grey curves analyzed with the Log Rank test on top and the Mantel cox test below.

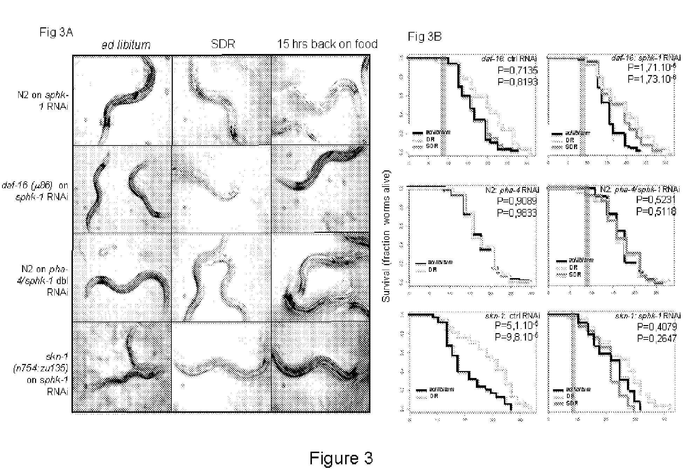

**Figure 3****:** Panel A on the right hand side shows the fat content of worms that were submitted to various diet regimen (as indicated on the graph) with *sphk-1* RNAi expressing bacteria as basic food. All worms were 2 days old when this experiment was performed so that accumulation of lipofuscin did not interfere with our analyses. When worms experienced a SDR, they were disposed on empty plates for 6 hours and then re-fed for 15 hours. Worm strains are specified on the graph. When worms were submitted to double RNAi against *pha-4* and *sphk-1,* the mixed was made with a ratio of 1:1. Panel B, on the left hand side, represent lifespan analyses as described previously (Kenyon et al, 1993) on agar plates containing 5-fluorouracil (15µM) to prevent growth of the progeny. In all panels, black curves represent control conditions where worms were fed *ad libitum* during their whole life. In contrast, light grey curves depict worms that were submitted to DR, performed by the method of food deprivation from day 1 of adulthood until then end of the experiment. Curves in dark grey represent the survival curves of worms that were submitted to a short dietary restriction (SDR) food deprivation in our system). A shaded area on the graph represents the period of the diet. Strains and conditions used are specified on the graph. Probability indicated on the graph concern the comparison between the black curves and the dark grey curves analyzed with the Log Rank test on top and the Mantel cox test below. When the dark grey curve is absent, comparison concerns the black and the light gray curves.

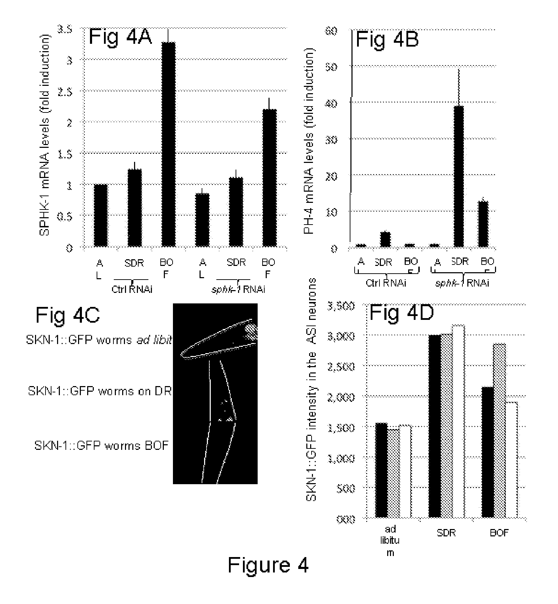

**Figure 4****:** Panel A shows expression levels of *SPHK-1* mRNA under various conditions specified on the graph. The experiment was performed on 2 days old worms. DR was imposed for 6 hours and RNA extracts were made. Finally, worms were put Back On Food (BOF) for 15 hours and new extracts were made for analyzes. All measurements were made by Q-PCR as described in Material and Methods. Panel B shows the expression level of the *PHA-4* gene under various conditions mentioned on the graph. The experiment was performed on 2 days old worms. DR was imposed for 6 hours and RNA extracts were made. Finally, worms were put Back On Food (BOF) for 15 hours and new extracts were made and analyzed. All measurements were made by Q-PCR as described in Material and Methods. Panel C illustrates the induction of SKN-1::GFP using the Is007 strain in the ASI neurons upon shift to DR for 4 days and their extinction after having returned to food for 24 hours. Panel D shows a quantification of the SKN-1::GFP signal in the ASI neurons in more conditions. Black columns represent *SKN-1::GFP* worms on the control vector (*ht115*)*.* Grey columns represents *SKN-1::GFP* worms on *sphk-1* RNAi. White columns represents *SKN-1::GFP* worms on *sphk-1* RNAi with addition of Sphingosine-1-Phosphate (5µM).

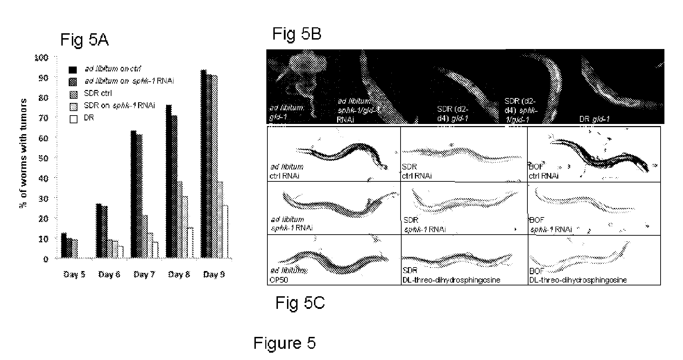

**Figure 5****:** Panel A shows the incidence of tumor as measured by the percentage of N2 worms that showed tumors in their gonads at the indicated time. The apparition of tumors was triggered by exposition of worms to *gld-1* RNAi. As indicated on the graph, worms were submitted to various diet regimens. Short Dietary Restriction (SDR) was imposed from day 2 to day 4 in order to not interfere with our analysis. When *sphk-1* RNAi was applied, it was mixed with *gld-1* RNAi in a ratio of 1:1. Panel B shows DAPI staining of whole animals at day 9 under specified conditions to illustrate the nature of the tumors observed. Panel C shows the effect of the chemical inhibitor of *SPHK-1,* DL-threo-dihydrosphingosine (130µM). Worms were 2 days old when the experiment was performed. Worms treated with DL-threo-dihydrosphingosine were treated at the time of SDR only. Worms fed *ad libitum* with the control bacterial strain (OP50) and concomitantly treated with DL-threo-dihydrosphingosine appear normal (not shown).

### DETAILED DESCRIPTION OF THE INVENTION

The approach of the inventors substantially differs from what has mostly been done. Instead of "forcing a complex metabolic response such as DR", they aimed at "boosting" and therefore "prolonging" an already existing DR response.

Indeed, in the course of their experiments, they noticed that an important property of the DR response is that it is rapid and reversible. In other words, short diets have no or little effects. Then, they looked for genetic determinants of this reversibility. Accordingly, their strategy consists in prolonging the positive metabolic effects of a short diet. They identified the sphingosine kinase as an essential regulator of DR reversibility. By inhibiting the sphingosine kinase, the inventors observed that the DR response becomes irreversible and its beneficial effect on longevity is retained.

Accordingly, the present invention concerns a therapeutic or non-therapeutic method for prolonging the benefits of a Dietary Restriction in a subject, comprising submitting the subject to a Dietary Restriction and administering to the subject an effective amount of an inhibitor of a sphingosine kinase during the Dietary Restriction and after the end of the Dietary Restriction. The present invention also concerns the use of a sphingosine kinase inhibitor for preparing a pharmaceutical or nutraceutical composition for prolonging the benefits of a Dietary Restriction. The present invention further concerns an inhibitor of a sphingosine kinase for prolonging the benefits of a Dietary Restriction.

In a first embodiment, the benefits of a Dietary Restriction are to slow down the ageing process in a subject or reduce the appearance of the effects of ageing. In particular, the benefits can be a decrease of the incidence of ageing related pathologies and an increased vitality and leanness. For instance, aging related pathologies can be selected from the group consisting of Amylotrophic lateral sclerosis (ALS), Alzheimer's Disease, Amyloidosis, Anal exudate, Angina pectoris, Anxiety, Arthritis (Osteoarthritis, Rheumatoid), Asthma, Atherosclerosis, Attention deficit disorder (ADD), Balance Disorders, Bell's Palsy, Blepharitis, Blood clots (thromboses), Cancer, Cataracts, Chalazion, Circadian cycle disturbances, Circulatory disturbances, Cold fingers, Cold legs, Colds, Congestive Heart Failure, Conjunctivitis, COPD, Corneal Abrasion, Coronary Artery Disease (CAD), Damages of pressure and stress, Dehydration, Dementia senilis, Depression, Diabetes Mellitus, Edemas, Embolisms, Emphysema, Eye Diseases, Failure to Thrive, Falls, Fatigue, Flu, Fungal infections, Generalized Anxiety Disorder (GAD), Glaucoma, Gums bleeding, Hemorrhoids, Hay fever, Headaches, Hearing Loss, Heart Disease, Heart Failure, High Blood Pressure, High Cholesterol, Hip Fracture, Immune system dysfunction (sensitivity to infectious bacterial and viral diseases), Internal bleedings, Liver damage, Liver inflammation, Loss of memory, Macular Degeneration, Memory Loss, Menopause, Migraines, Multiple sclerosis (MS), Neck Fracture, Night blindness, Osteoarthritis, Osteoporosis, Parkinson's Disease, Period disturbances, Phlebitis, Pneumonia, Prostate disturbances (e.g. enlargement, cancer), Psoriasis, Retinopathy, Rheumatism, Rheumatoid Arthritis, Sarcopenia, Sexual dysfunction, Sores, Spinal Stenosis, Stroke, Substance Abuse, Swollen joints, Swollen veins, Urinary Incontinence, and Vertebral Fracture.

In a second embodiment, the benefits can be a decrease of the body mass, in particular of the body fat content. Accordingly, the sphingosine kinase inhibitor can be useful in order to prevent or treat obesity and related metabolic disorders. Obesity shall be construed as any condition of abnormal or excessive fat accumulation in adipose tissue, to the extent that health may be impaired. Associated disorders, diseases or pathologies include, more specifically, any metabolic disorders, including diabetes mellitus (more particularly type II diabetes) and associated complications such as diabetic neuropathy, hypo-alphalipoproteinemia, familial combined hyperlipidemia, hyperinsulinemia, insulin resistance, insulin resistant syndrome X or multiple metabolic disorder, cardiovascular complications such as coronary artery disease, and dyslipidemia.

However, the decrease of the body mass can also be beneficial for esthetical or comfort purposes and not only for therapeutic ones. Such a use of sphingosine kinase inhibitors is also contemplated by the present invention.

Where "about" is used in connection with a number, this preferably means the number +/-15%, more preferably the number plus 5%, most preferably the number itself without "about".

By "effective amount" is intended the amount of drug that allows the maintenance of the subject in a physiologic state established by the Dietary Restriction. In order to ensure that a subject is effectively under a "DR metabolic state", several parameters can be assessed. Biological parameters can be assessed such as mitochondrial morphology, expression levels of DR indicators like the AMP kinase, glucosc-6-phosphate isomerase, fructose 1,6-bisphosphatas , IPP-2, and transketolase, fatty acid synthase, PPAR-delta, glutamine synthase, purine nucleoside phosphorylase, thymidylate kinase, elongation factor 1-gamma, proteasome activator PA28, translocon-associated protein delta, 60S ribosomal protein L23, the 26S proteasome subunit TBP-1 Other genes are reduced during DR such as DnaJ homolog, inducible heat shock, cytochrome P450 isoforms IIIA and Cyp1b1, Hsp105, aldehyde dehydrogenase, XPE, RAD50, DNA polymcrase-beta and the thyroid-hormone receptor alpha gene (Lee et a1, Science, 1999 Vol. 285. no. 5432, pp. 1390 - 1393). Other physiological indicators can also be assessed such as decreased of body temperature, decreased levels of plasma insulin, fasting glucose, IGF-1, lL-6, IL-10, oxidative damage to muscle, glycation products, serum triglycerides, lipoproteins, growth hormones. Lower systolic blood pressure, heart, rate, higher insulin sensitivity, higher serum HDL2B, higher levels of interferons-γ, higher proliferative capacity of fibroblast and higher acoustic responses are also seen. Finally, we note increased levels of DHEAS (Roth et al, Science 2002, Vol. 297. no. 5582, p, 811) and of triiodothyronine (Roth et al, Science 305, 1423 (2004) ; Roth et al, Horm Metab Res. 2002 Jul;34(7):378-82).

The sphingosine kinase inhibitors can be any molecules able to decrease the enzyme activity, either by a direct effect on the sphingosine kinase activity or by an indirect effect through a decrease of the expression of the sphingosine kinase.

Reference to "sphingosine kinase" should be understood as reference to all forms of this protein and to functional derivatives and homologues thereof. This includes, for example, any isoforms which arise from alternative splicing of the subject sphingosine kinase mRNA or functional mutants or polymorphic variants of these proteins. For example, this definition extends to the isoforms sphingosine kinase-1 and sphingosine kinase-2. In a preferred embodiment, the sphingosine kinase is the sphingosine kinase-1. A " sphingosine kinase-1 ", "SK1", "SphK1", or "SK1 protein" each refers to a protein that upon activation is capable of catalyzing the formation of sphingosine- 1 -phosphate (SIP) from the lipid sphingosine. The sphingosine kinase 1 gene is referenced on NCBI HomoloGene as id 39748. A " sphingosine kinase-2 ", "SK1", "SphK2", or "SK2 protein" each refers to a protein having the same function than the sphingosine kinase 1. The sphingosine kinase 2 gene is referenced on NCBI HomoloGene as id 32456.

Molecules suitable to decrease the expression of a gene are well-known by the man skilled in the art. For instance, the inhibitors can be an antisense DNA, an antisense RNA, a ribozyme or a small interfering RNA specific of the gene encoding the sphingosine kinase. Suitable siRNA molecules are disclosed in the patent applications WO2008/067560 and WO2008/067526. In a specific embodiment, the inhibitors can be the siRNA disclosed in the Examples section.

Other ways to decrease the activity of the sphingosine kinase are disclosed in the patent applications WO 2006/135969 and WO 2006/135968 by preventing the interaction of the sphingosine kinase with other proteins activating the sphingosine kinase (the disclosure of which are incorporated herein by reference).

Alternatively, the sphingosine kinase inhibitors are chemical compounds, such as the well-known compounds N,N-dimethylsphingosine, or DL-threo-dihydrosphingosine. However, other suitable compounds are disclosed in the patent applications WO 2007/019251, WO 2006/138660, US2006/270630, WO 2006/004359, WO 2005/118521, Kim et al ((2005) Bioorganic & Medicinal Chemistry, 13, 3475-3485, e.g. Table 1) and WO 2003/105840 (the disclosure of which are incorporated herein by reference). Other described inhibitors are as follow: erythro-enantiomers of sphingosine and dihydrosphingosine, F-12509A : sesquiterpene quinone consisting of a drimane moiety and a dihydroxybenzoquinone (Kono et al, J Antibiot (Tokyo). 2000 May;53(5):459-66), B-5354c, the long unsaturated aliphatic chain, 4-amino and 3-hydroxyl groups (kono et al, J Antibiot (Tokyo). 2000 Aug;53(8):759-64), B-535a, b and c : esters of 4-amino-3-hydroxybenzoic acid with long-chain unsaturated alcohols (kono et al, J Antibiot (Tokyo). 2000 Aug;53(8):753-8) S-15183a and b :new azaphilone-type métabolites (kono et al, J Antibiot (Tokyo). 2001 May;54(5):415-20).

Otherwise, the sphingosine kinase inhibitors can be a sphingosine kinase dominant negative such as SphK^{G82D} (WO 2006/135967 and WO 2006/135967, the disclosure of which are incorporated herein by reference).

The activity of the sphingosine kinase can be decreased at various degrees. For instance, the activity of the sphingosine kinase has to be decreased by at least 10, 15, 20, 25, 30, 35, 40, 45 or 50 %. Alternatively, the activity of the sphingosine kinase has to be decreased by less than 10, 15, 20, 25, 30, 35, 40, 45 or 50 %. Optionally, the activity of the sphingosine kinase has to be decreased from 10 to 20, 30, 40, 50, 60, 70, 80, or 90 %, from 20 to 30, 40, 50, 60, 70, 80, or 90 %, from 30 to 40, 50, 60, 70, 80, or 90 %, or from 40 to 50, 60, 70, 80, or 90 %. The activity of the sphingosine kinase can be assessed by any method known in the art. For instance, a sphingosine kinase activity can be assessed with purified enzyme in presence of the test compound through the rate of sphingosine phosphorylation. Alternatively, following treatment with a test compound, cells can be incubated with [³H]sphingosine at a final concentration of 1 µM, and then, the [³H]S1P amount is determined (French et al, 2003, Cancer Res 63, 5962).

Accordingly, the effective amount of sphingosine kinase inhibitors can be adapted to reach such a percentage of inhibition.

The sphingosine kinase inhibitor can be formulated as a pharmaceutical composition, a beverage, a food, a feed, a nutraceutical, a dietary supplement or a functional food. Accordingly, the present invention also relates to a method for preparing a beverage, food, feed, nutraceutical, dietary supplement or functional food comprising adding the sphingosine kinase inhibitor to at least one component selected from the group consisting of beverage ingredients, food ingredients, animal feed ingredients, pet food ingredients, nutraceutical ingredients, dietary supplement ingredients, and functional food ingredients.

The pharmaceutical composition further comprises any convenient pharmaceutically acceptable carrier. In a particular embodiment, the composition is suitable for oral route or for intravenous, intra-tumoral or sub-cutaneous injection, or for intracranial or intra artery injection or infusion or for topic administration.

The subjects can be an animal, preferably a mammal, or a human being. For instance, the animal can be for instance a cat, a horse or a dog. The subjects are preferably humans, i.e., men and women. The subjects are adults. In particular, the subject is a middle aged subject or an elderly. By "middle aged" is intended between about 35 and about 65 year-old subject for humans, preferably between about 40 and about 60. By "elderly" is intended a subject being at least 60 year-old for humans, preferably 65 year-old.

The subject is submitted in a first phase to a Dietary Restriction. In the art, Dietary Restriction can also be called Calory or Caloric Restriction. During the Dietary Restriction phase, energy intake is minimized, but sufficient quantities of vitamins, minerals and other important nutrients must be eaten. For instance, during the Dietary Restriction phase, the dietary regimen comprises 20, 25, 30, or 35 % less calories than the standard regimen. In particular, for a woman, the dietary regimen can comprise between 800-1200 kcal/day and, for a man, between 1200-1500 kcal/day.

The phase of Dietary Restriction is rather short. For instance, this phase is a period of time of one, two, three or four weeks, or one, two, three, four, five or six months. In particular, this phase can be less than, six, five, four, three, two or one months.

The sphingosine kinase inhibitor is administered to the subject during the phase of Dietary Restriction and after the end of this phase. Preferably, the sphingosine kinase inhibitor is administered during all the Dietary Restriction (e.g., since the beginning and until to the end). In particular, after the end of this Dietary Restriction phase, the sphingosine kinase inhibitor is administered to the subject as long as it allows the prolongation of the Dietary Restriction benefits. For instance, the phase of the administration of a sphingosine kinase inhibitor without any Dietary Restriction can be one, two, three, four, five or six months. The end of the prolongation can be optionally assessed by the determination of a parameter of the Dietary Restriction effect. For instance, this parameter can be selected from the group consisting of the expression of PHA-4 homolog and/or mitochondria morphology. In order to ensure that a subject is effectively under a "DR metabolic state", several parameters can also be assessed. Biological parameters that can be assessed are the following expression levels of DR indicators like the AMP kinase, glucose-6-phosphate isomerase, fructose 1,6-bisphosphatas , IPP-2, and transketolase, fatty acid synthase, PPAR-delta, glutamine synthase, purine nucleoside phosphorylase, thymidylate kinase, elongation factor 1-gamma, proteasome activator PA28, translocon-associated protein delta, 60S ribosomal protein L23, the 26S proteasome subunit TBP-1, Other genes are reduced during DR such as DnaJ homolog, inducible heat shock, cytochrome P450 isoforms IIIA and Cyp1b1, Hsp105, aldehyde dehydrogenase, XPE, RAD50, DNA polymerase-beta and the thyroid-hormone receptor alpha gene (Lee et al, Science, 1999 Vol. 285. no. 5432, pp. 1390 - 1393). Other physiological indicators can also be assessed such as decreased of body temperature, decreased levels of plasma insulin, fasting glucose, IGF-1, IL-6, IL-10, oxidative damage to muscle, glycation products, serum triglycerides, lipoproteins, growth hormones, Lower systolic blood pressure, heart rate, higher insulin sensitivity, higher serum HDL2B, higher levels of interterons-γ, higher proliferative capacity of fibroblast and higher acoustic responses are also seen. Finally, we note increased levels of DHF AS (Roth et al, Science 2002, Vol. 297. no. 5582, p. 811) and triiodothyronine (Roth et al, Science 305, 1423 (2004) ; Roth et al, Horm Metab Res. 2002 Jul;34(7):378-82 ). In particular, a high expression level of the human PHA-4 ortholog (Forkhead box Al gene ; NCBI accession number NP_004487.2) is indicative of a Dietary Restriction state. In addition, punctuated muscle mitochondria with a lower degree of interconnection is also indicative of a Dietary Restriction state.

In a particular embodiment, the cycle of the first and second phases can be repeated. Accordingly, another Dietary Restriction phase with a treatment with a sphingosine kinase inhibitor is applied followed by the treatment with the sphingosine kinase inhibitor without the Dietary Restriction. The cycle can be repeated once, twice, etc... In particular, the cycle is repeated as long as it can be tolerated and up to the death of the subject.

### Screening method

The present invention also concerns a method for identifying or screening a molecule suitable for prolonging the benefits of a Dietary Restriction. The method is based on the use of *C. elegans* worms. The use of *C. elegans* as a genetic model for studying Dietary Restriction has been validated in the art. The method comprises 1) providing adult worms with a normal fat content; 2) submitting the worms to food deprivation during about 4-7 hours in the presence of a test molecule; 3) cultivating the worms in an axenic medium containing the test molecule during about 15-25 hours; 4) determining the fat content of worms; and, 5) selecting the test compound if the fat content of worms is low, said selected compound being suitable for prolonging the benefits of a Dietary Restriction. By normal fat content is intended the fat content of a worm when fed at will. The above screening assay may be performed in any suitable device, such as plates, tubes, dishes, flasks, etc. Typically, the assay is performed in multi-well plates. Preferably, the assay is carried out in a 96 well plate. Several test compounds can be assayed in parallel. Furthermore, the test compound may be of various origin, nature and composition. It may be any organic or inorganic substance, such as a lipid, peptide, polypeptide, nucleic acid, small molecule, etc., in isolated or in mixture with other substances. The compounds may be all or part of a combinatorial library of products, for instance. Preferably, the worms are *C. elegans* or *Caenorhabditis briggsae,* more preferably *C*. *elegans.* However, the present invention also contemplates a method of screening using any animal on which a DR parameter can be detected easily. Indeed, the selection criterion is a delay in return to the normal metabolic state.

The adult worms provided in step 1) present a normal fat content. For instance, they can be about 3 day-old. They can be provided as a suspension in a buffer. When the method is carried out in a 96 well plate, 10-30 worms can be distributed into each well, preferably about 20 worms.

The worms are submitted to a food deprivation in the presence of a test molecule during a period of about 4-7 hours, preferably about 6 hours. During the food deprivation, no nutrient is available for the worms. The worms are in a buffer containing the test molecule, e.g., a phosphate buffer. During this deprivation phase, the worms reach a low fat content. The deprivation step is sufficiently long to obtain worms with a low fat content.

After the deprivation step, the worms are cultivated in an axenic medium containing the test molecule during between about 15 to about 25 hours, preferably between about 18 and about 22 hours, more preferably about 20 hours. The axenic medium can comprise yeast extract, peptones, hemoglobin and/or the likes.

In a first embodiment, the step 4) can be performed by the worms' observation under a microscope. The observation is based on the presence of fat near the intestine. Indeed, the more they have fat, the more they are dark. On the opposite, the less they have fat, the more they are clear. Accordingly, a worm with a normal fat content is dark and a worm with a low fat content is clear. In order to avoid any subjectivity in the assessment of the dark and clear criteria, the method comprises the use of control worms. In particular, the clear control worm is a worm submitted to a deprivation for at least 6 hours and the dark control worm is a worm cultivated in a medium comprising soy peptones, yeast extract and hemoglobin. Any medium supporting growth and ends the DR regimen that animals just experienced will work as long as it does not interfere with scoring of the DR specific trait that is being assayed. For example, fat accumulation cannot be assayed with medium containing bacteria as it blurs the medium out and detection is rendered impossible. In a preferred embodiment, the selection is based in the criteria of the darkness or clarity of worms which is easy to discriminate.

In a second embodiment, the step 4) can be performed automatically thanks to a large particle FACS machine (e.g., COPAS BIOSORT). Examples of parameters allowing the discrimination between a worm with a normal fat content and a worm with a low fat content are the optical density and the autofluorescence (in green and/or in yellow).

The method also contemplates additional observation before and/or after the end of the period of 15-25 hours. For instance, the worms can be observed every five or ten hours.

The method can further comprise the determination of other parameters such as the worms lifespan, the mitochondria morphology, the presence of tumors in worms, the oxygen consumption, protein aggregation, dynamicity, induction level of a PHA-4::GFP transgene, induction levels of a SKN-1::GFP transgene, induction levels of any significant gene significantly induced by DR, size, etc...

The example illustrates the invention without limiting its scope.

### EXAMPLES

### Example 1 : Shingosine kinase inhibitors prolong the benefits of DR

Longevity is closely linked to food intake. Indeed, both quality and quantity of the food that is ingested can affect longevity. Excessive or unbalanced food consumption causes various morbid diseases such as obesity and/or diabetes but defining the "optimal" food intake for health remains a challenging task today. McCay showed in 1935 that rats fed at will did not eat optimally with respect to their longevity spontaneously (McCay et al., 1935). Rather, it seems that voluntary feeding is optimized for reproduction. One way to interpret this data is that food shortage increases lifespan so that individuals can "wait longer" for more favorable conditions that will augment the chances to properly transmit genetic material to their progeny (McCay et al., 1935). In any case, diminishing daily food intake without reaching malnutrition extends both lifespan and the reproductive period and this manipulation, called Dietary Restriction (DR), is reproducible in all species tested so far, although evolutionarily divergent (Mair and Dillin, 2008). This comforts the view (1) that long life is not a trait that is selected for *per se* since food intake has obviously not been optimized for longevity (Kirkwood, 2005) (2) and that food shortage can increase lifespan most likely to improve reproductive success. The long term effects of DR (including lifespan extension) has not yet been formally tested on humans but the metabolic response to diminished food intake seems comparable in all point to what was observed in other animals (Fontana et al., 2004; Walford et al., 1999; Weyer et al., 2000). The conservation of this response validates the use of short-lived genetic models such as worms, flies or even yeast and therefore eases fast scientific investigation of its biological properties. In fact, all genetic actors of DR and therefore, chemical agents, such as resveratrol, developed to mimic DR were first discovered in yeast or nematode (Baur and Sinclair, 2006). Recently, two *bona fide* genetic regulators of the DR response were identified using the roundworm *Caenorhabditis elegans:* PHA-4 and SKN-1b (Bishop and Guarente, 2007; Panowski et al., 2007). Both these genes have orthologues in humans and it is therefore presumed that their role in mediating DR could be conserved. PHA-4 is a forkhead transcription factor that is required to generate the pharyngeal primordium during development and to mediate the DR response during adulthood (Mango et al., 1994; Panowski et al., 2007). It is homologous to the FOXA transcription factors that also fulfill metabolic functions in mammal since they protect mice against hypoglycemia by promoting the expression of proglucagon in pancreatic b cells (Friedman and Kaestner, 2006). SKN-1 is a leucine zinc finger transcription factor, which is involved in the fate of ventral blastomeres as well as in the oxidative stress response in worms (An and Blackwell, 2003; An et al., 2005; Bowerman et al., 1992). It is homologous to the NF-E2-related transcription factors (Nrf1 and Nrf2; (Walker et al., 2000)) that are also important for the oxidative stress response in mammals (Li et al., 2007). Interestingly, Nrf2 is responsible for the anticarcinogenic effects of DR in mice (Pearson et al., 2008). PHA-4 and SKN-1b are therefore two transcription factors necessary to the DR response and their expression levels, but not their localization, respond to a DR treatment (Bishop and Guarente, 2007; Panowski et al., 2007). Although it has not yet been tested, it is possible that PHA-4 and SKN-1 interact. Because PHA-4 is constitutively expressed in the nuclei of the intestine, the head and the gonad and the b isoform of SKN-1 is in the nuclei of the nutrient sensing neurons (ASI), it was suggested that this SKN-1b should be upstream PHA-4 and that the interaction should be indirect and potentially mediated by hormones (Antebi, 2007).

Intriguingly, although the inventors have known for long that DR lowers the incidence of all aging related pathologies, including cancer, diabetes and neurodegenerative diseases and is associated with increased youthfulness (Masoro, 2002), this simple manipulation is still not widely performed by humans. Part of the explanation of this apparent paradox may lie in the observation that the few human beings who have tried to apply DR on themselves noticed important deleterious psychological consequences such as depression, irritability and social isolation probably triggered by the continuous feeling of hunger (Dirks and Leeuwenburgh, 2006). Other reasons can simply be that few people are actually willing to modify their eating habits. The inventors report here on a new strategy to efficiently benefit from DR without its adverse side effects consisting in modifying its temporal requirements at the molecular level. Indeed, DR can be successfully imposed late in life (but it then only concern few individuals; (Smith et al., 2008)) but short diets do not suffice to extend lifespan. Thus, the DR state is reversible. The inventors reasoned that if they could find genetic determinants of DR reversibility, they could prolong the DR response. They show in the present invention that the sphingosine kinase (SPHK-1) mediates the reversibility of DR. Thus, when coupled to a short DR treatment (SDR), genetic or chemical deactivation of the SPHK-1 gene leads to increased lifespan and long lasting of most of the DR associated phenotypes, including leanness. Mechanistically, they show two interesting features of the SPHK-1. First, its expression is induced at the time of re-feeding (after a DR period), consistently with a role in DR reversibility. Second, the inhibition of SPHK-1 coupled to short DR boosts the induction of PHA-4 triggered by DR. Thus, this effect is fully dependent on the presence of the DR master regulator, PHA-4. The inventors show that the presence of SKN-1 is also required although the interaction between SKN-1 and SPHK-1 seems less direct. On the other hand it is independent of DAF-16, suggesting that it specifically prolongs a DR response. Finally, the inventors show that transient DR treatment can delay the onset of tumor formation only when SPHK-1 is partially deactivated.

In summary the inventors indentify the inhibition of SPHK-1 has a powerful way to prolong a short DR. In contrast to other attempts to mimic DR such as resveratrol administration, the proposed strategy does not aim at "faking" a metabolic state but rather prolonging an existing state. The inventors also believe that their approach is more realistic because it is less invasive. Indeed this strategy does not interfere with important metabolic responses such as appetite (like ghrelin antagonists).

### The Dietary Restriction response is reversible

It was recently suggested that DR does not have specific temporal requirements (Smith et al., 2008). Indeed, the Kaeberlein lab showed that, in worms, DR could be successfully imposed late in life (until day 24). They also demonstrated that a 8 days DR performed early in life led to a significant lifespan extension although much less pronounced than a DR of 14, 20 or 28 days (Smith et al., 2008). Smith at al., showed that a 4 days DR, performed between day 4 and day 8 of adulthood, slightly increased heat resistance at day 12 but lifespan data was not measured under these conditions to verify whether this effect sufficed to prolong lifespan. First, the inventors confirmed this data by analyzing the lifespan of worms that were switched to DR (using the bacterial deprivation protocol) at day 10 of adulthood until their death. This manipulation triggered a lifespan extension similar to what is observed when worms are submitted to DR during their whole adult life (Figure 1A; (Smith et al., 2008; Sutphin and Kaeberlein, 2008)). The inventors then performed the opposite experiment by submitting worms to a DR (bacterial deprivation) form day 1 to day 10 of adulthood and then switching them back on to a normal regimen. As shown in Figure 1B, the inventors observed that lifespan of these worms was slightly lengthened although to a much lesser extent than what was seen in the first experimental set up (late diet). This in line with data published in *Smith et al* (Smith et al., 2008). Figure 1B clearly shows that that the DR response did not last indefinitely. This prompted them to hypothesize that worms quickly adapted to their environment and that a "DR state" was only reached at the time of the DR treatment. In order to critically test this hypothesis, the inventors performed Short Dietary Restriction (SDR) of 2 days at various time points between day 1 and day 10. SDRs were performed at day 2, 5 or 8. In none of these cases did the inventors observe a lifespan increased (Figure 1C, 1D, 1E). They conclude that the DR response is reversible, reconciling worms and flies data (Mair et al., 2003). Indeed demographic studies performed on *Drosophila melanogaster* suggested that lower mortality rate was only measured on animals actually experiencing a DR treatment (Mair et al., 2003). Interestingly, when flies were put back on a full regimen diet, their mortality rate instantly increased, suggesting that the biological state of DR could be acquired rapidly and was fully reversible. The inventors speculate that the reversible character of the DR response is conserved.

### RNA interference against the sphingosine kinase hinders fat re-accumulation after a short dietary restriction

In order to screen for genes that mediate the reversibility of the DR response, the inventors used the simplest phenotype associated to SDR: fat content. Indeed, in many organisms including worms, the most readily observable phenotype that accompanies a decrease in food intake is fat loss (Figure 2A). Since nematodes are essentially transparent, lipids contained in intestinal cells appear darker because of their high density. Thus, they can easily be monitored under a binocular dissecting microscope. Indeed, the intestinal compartment in worms also plays the role of fat storage organ. To avoid potential misinterpretations caused by the age related darkening of worms caused by lipofuscin accumulation, the inventors chose to perform their screen at day 2 of adulthood. At this time, lipofuscin has not yet appeared and does not interfere with the analysis. When fed *ad libitum,* worms are dark and they turn transparent within 6 hours of food deprivation ((McKay et al., 2003) and Figure 2A). At this point, the inventors switched animals back to an *ad libitum* regimen and monitored the amount of fat re-accumulated after 15 hours. Control animals are as dark as worms that never experienced SDR (Figure 2A). The inventors screened for genes that could affect fat re-accumulation only. In other words, they looked for clones that did not affect fat content either before or during DR but only after having been returned to control diet (Figure 2A). They restricted their screen to molecules most likely to be involved in food signalling (such as kinases) or in metabolic transcriptional response (nuclear hormone receptors). The inventors found 6 positive clones amongst which the clone *C34C6.5* which is directed against the only worm homolog of the sphingosine kinase, SPHK-1. As shown in Figure 2A, worms fed with bacteria expressing double stranded RNA interfering with SPHK-1 were undistinguishable at first sight from worms treated with the empty vector strain when fed *ad libitum* or under DR. Hence, they could make and lose fat normally. However, they were clearly deficient in re-gaining fat and therefore appeared clearer than wild type 15 hours after re-feeding (see Figure 2A). Importantly, fat re-accumulation was not fully stopped in these animals as they ultimately did re-gain fat on a control diet (see Figure 2A). This reinforced the impression that RNAi against *SPHK-1* prolonged the leanness associated to DR but did not lock the animals in a given metabolic state.

Importantly, *SPHK-1* is located on chromosome II and is in an operon that contains 2 genes: *SPHK-1* and the choline dehydrogenase (C34C6.5). Since it is formally possible that RNAi directed against C34C6.5 could also inhibit C34C6.4, the inventors performed chemical and genetic inhibition of the two proteins to test to which of the two genes was indeed involved in fat re-gaining deficient. First, RNAi against C34C6.4 did not show any obvious phenotype and importantly treatment with a chemical inhibitor of the choline dehydrogenase (3,3-dimethyl-1-butanol) increased the motility of the worms (data not shown), confirming the efficacy of the drug, but fail to produce any fat re-accumulation phenotype. On the other hand, DL- threo-dihydrosphingosine that can inhibit SPHK-1 can recapitulate the phenotype (See Figure 5C). Finally, the fat-reaccumulation phenotype observed under RNAi condition is abolished by addition of sphingosine-1-phosphate (S1P) directly on the plate (Figure 2A). Although, the existence of an operon suggests a meaningful co-regulation of the two genes, the inventors conclude the SPHK-1, but not the choline dehydrogenase, is responsible for the prolongation of the leanness associated to DR. In addition, it does so by modulating the levels of S1P.

This finding clearly constitutes a novel way to slow down fat accumulation after a diet and can be used as a base for anti-obesity drug. The inventors reasoned that inhibiting this gene might allow a metabolic memory of a past diet and hoped that it could prolong other beneficial effects associated with the DR response.

### Inhibiting the sphingosine kinase prolonged most of the DR associated phenotypes, including extended lifespan

Next, the inventors set out to analyze phenotypes associated to RNA interfering with *sphk-1.* Because of its role in the control of cell proliferation in mammalian (Spiegel and Milstien, 2007), they first studied the state of the only proliferative cells in the worm: the gonad. By simply staining worms with DAPI and counting nuclei in the gonad, they could observe a slight effect in the position of the transition between the mitosis and the meiosis zones, as evidenced by the presence of nuclei with a mitotic specific morphology (dense crescent shape) in the area of the gonad where meiosis usually occurs: the pachytene (Figure 2B). Thus, as in mammals, the worm sphingosine kinase regulated cell proliferation. This was expected and confirmed the identity of the kinase inhibited. Importantly, the RNAi treatment did not reduce fertility suggesting that the imposed inhibition was small. Indeed, it is known that the complete abolition of the sphingosine kinase activity is lethal (Mizugishi et al., 2005). Also, animals with similar germline defects (Figure 2B, fbf-1 and fbf-2 RNAi; (Lamont et al., 2004)) did not share the fat re-accumulation phenotype, indicating that *SPHK-1* plays a role in both cell proliferation and fat re-accumulation but that these two phenotypes can be regulated distinctly.

In order to further explore the possibility of having found a gene that mediated the reversibility of a metabolic state associated to DR, the inventors systematically investigated characteristics of animals that were both treated with sphingosine kinase RNAi and put on a short DR (SDR) upon their return to an *ad libitum* diet (Short *D*ietary *R*estriction+*sphk-1* RNAi: SDR+S treatment).

A series of phenotypes could be observed to be associated to SDR. As described just above the most obvious phenotype, fat loss, could be prolonged by a SDR+S treatment (Figure 2A).

Because of the intuitive metabolic feature of the phenotype revealed by the present screen, the inventors looked at the mitochondria morphology of animals on DR versus animals fed *ad libitum.* Strikingly and as had already been shown in flies (Magwere et al., 2006), they observed a drastic re-organization of mitochondria morphology upon diet shifts. Using both MYO-3::GFP tagged animals that express a GFP signal in the mitochondria of the muscle and the general mitochondria stain DiOC6, they observed that under and *ad libitum* diet, mitochondria were filamentous and highly interconnected (Figure 2C). In contrast, when worms were shifted to a short restricted diet (SDR), muscle mitochondria clearly became punctuates and the degree of interconnection was much lower (Figure 2C). Using DiOC6, the inventors noticed that the punctuate morphology could be observed both in the muscle and in the gonad (Figure 2C and data not shown). When the inventors switched animals back to an *ad libitum* diet, they observed that mitochondria became interconnected again after 15 hours exposure to unlimited amount of food (Figure 2C). On the other hand, when worms had gone under the SDR+S treatment, their mitochondria remained punctuated after return on food for 15 hours (Figure 2C). Thus, inhibition of *sphk-1,* in addition of delaying fat re-accumulation after return to food also impedes return to mitochondrial morphology usually associated to an *ad libitum* diet. Rather animals stayed in a DR like state. The inventors believe that this argues in favour of SPHK-1 as being a modulator of the reversibility of the DR response. Altogether, the present results suggest that the SDR+S treatment really prolong the positive effects of DR. This prompted the inventors to verify whether inhibition of *sphk-1* could prolong DR induced longevity altogether. They then set out to measure the lifespan of animals that had been submitted to a *sphk-1* RNAi treatment from hatching and that also were put onto SDR at various time points during their life history. When animals went through a diet at early stages of their adult life (before or while they reproduced), it was detrimental to their longevity and *sphk-1* RNAi treatment did not affect this much at all (data not shown). When SDR was imposed after the reproductive period and prior to the beginning of the natural dye off of the worms (between day 8 and day 10), it had no effect on worms treated with the empty vector but inhibition of *sphk-1* allowed a longevity extension comparable to that of animals that are on permanent DR (Figure 2D). The inventors conclude that SPHK-1 mediates the reversibility of restricted diet induced longevity.

### PHA-4 and SKN-1 but not DAF-16 are required for lifespan extension by the SDR+S treatment

To elucidate the mechanisms of action of SPHK-1, the inventors tested whether known longevity genes could interact with SPHK-1. First, they concomitantly deactivated *sphk-1* and other longevity genes (see table 1). As shown in figure 3A, they found that three genes could efficiently suppress the fat re-accumulation phenotype provoked by a SDR+S treatment: *PHA-4, SKN-1* and *DAF-16.* This came as a surprise because although PHA-4 and SKN-1 are known regulators of the DR response, DAF-16 is not. However, it was very recently suggested that DAF-16 fulfilled lipogenic functions and that this is independent of its role in regulating longevity (Perez and Van Gilst, 2008). It is formally possible that, although fat loss and DR are usually associated, they can be dissociated at the molecular level and that fat content is also under the influence of the insulin pathway. To clarify this issue, the inventors went on to test the potential suppressor effect of PHA-4, SKN-1 and DAF-16 of the longevity phenotype triggered by the SRD+S treatment. Figure 3B shows that, as expected, PHA-4 and SKN-1 are fully necessary to the SDR+S mediated lifespan but that the absence of *daf-16* did not affect this response. This data clearly situates *sphk-1* in the DR pathway and not in the IIS pathway. The inventors conclude that inhibiting *sphk-1* and thereby lowering the amount of S1P, specifically prolongs the DR response.

### SPHK-1 is activated at the end of Dietary Restriction and its inhibition boosts the PHA-4 transcriptional response to DR

The inventors measured the mRNA levels of *SPHK-1* upon various conditions. As shown in figure 4A, the expression of *SPHK-1* was unaffected by DR but induced upon return to food, consistently with a role in the reversibility of the DR response. As expected, *sphk-1* RNAi hindered this response but it is important to note that the degree of inhibition it triggered remained small. This is in accordance with the classical view that S1P levels must be maintained at a certain levels. Indeed, mice lacking sphingosine kinase activity are not viable and *C. elegans* mutants for *sphk-1* seem lethal as well (data not shown). PHA-4 and SKN-1 are two transcription factors that were recently reported to be essential to the DR response since DR is ineffective in their absence. Interestingly, they are responsive to DR since both mRNA levels of PHA-4 and SKN-1::GFP signal in the ASI neurons increase upon shift to DR. The inventors measured these parameters when *sphk-1* was knocked down by RNAi. In figure 4B, it is shown that PHA-4 mRNA levels, measured by Q-PCR, increased 2 folds upon switch from control to DR conditions but 39 fold when *sphk-1* was inhibited. Consequently, the return of PHA-4 levels to normal after returning to *ad libitum* diet conditions took longer under these conditions. The inventors propose that this accounts for the DR prolonging effect of *sphk-1* inhibition. Because Bishop and Guarente showed that the levels of the b isoform of the SKN-1 gene was also induced by DR, they used SKN-1::GFP worms to measure the effect of *sphk-1* inhibition on this induction. On Figure 4C and 4D, it is shown that (i) SKN-1 is indeed induced in the ASI neurons upon DR treatment and that (ii) this induction is not affected by the inhibition of *sphk-1.* However, when worms were returned to an *ad libitum* diet for 24 hours, the SKN-1::GFP signal decreased significantly less rapidly for worm fed *sphk-1* RNAi suggesting that the return to normal metabolic conditions was slowed down (Figure 4D). Interestingly, this retard was fully abrogated by addition of S1P, confirming a central role for this molecule in the reversibility of the DR response (Figure 4D). Importantly, *sphk-1* RNAi could not prolong DAF-16 nuclear localization triggered by DR (data not shown). Indeed, although DAF-16 goes nuclear under DR but it is known to not play a role in its longevity-promoting role. Thus, the present data suggests that *sphk-1* RNAi specifically prolong the DR response by boosting both the transcriptional response of PHA-4 to DR and by retarding the decrease of SKN-1 levels in the ASI neurons upon return to normal feeding conditions.

### The SDR+S treatment delays tumor formation on gld-1 animals

It seemed important to us to specifically test the effect of the SDR+S treatment on age related diseases such as cancer that constitutes major health issue in western civilizations today. *C. elegans* is a post mitotic organism and usually do not develop tumors. However a simple manipulation such as RNAi against the *gld-1* gene leads to the apparition of tumors within the only dividing tissue of the animal: the gonad. Interestingly, it was already reported that *eat-2* mutant animals (a DR surrogate) resist to the age-associated apparition of tumors in *gld-1* RNAi treated animals (Pinkston et al., 2006). Although sphingosine kinase inhibitors have already been proposed as anti-carcinogenic agents, the inventors show here that the level of inhibition of *sphk-1* triggered by RNAi does not suffice to lower tumors formation (Figure 5A, and 5B). SDR alone performed between day 2 and day 4 of adulthood has a short lasting effect on tumor formation since worms that experienced SDR have as many tumors as worms that did not by day 9. On the other hand, the SDR+S treatment allowed a long lasting amelioration of the *gld-1* mediated tumor phenotype (Figure 5A). The inventors conclude that the combination of a SDR with slight inhibition *sphk-1* markedly decreases age related tumor formation. They propose that the SDR+S treatment could constitute an efficient strategy to diminish the occurrence of age related cancers without lower doses of drugs.

### Chemical inhibition of Sphingosine kinase also prolong the DR response

The final set of experiments of this study concern the use of chemical agents to potentially mediate a DR prolonging effects. There already exist a large number of known inhibitors of Sphingosine kinase described in the literature (Buehrer and Bell, 1992; Delgado et al., 2006; Kim et al., 2005) because of their recognized effect on cell proliferation by altering the balance between Sphingosine and Sphingosine-1-Phosphate (Hait et al., 2006). However, none of these drugs have been reported to have an effect on either fat accumulation or aging. Safingol or DL-threo-dihydrosphingosine is one such commercial inhibitor. When the inventors administered Safingol on plate containing wild type animal at 200µM, they observed that worms lost their fat drastically and appeared sick (data not shown). They therefore diminished the concentration of Safingol administered until no apparent phenotype could be observed. Satisfactorily, when they imposed a short diet of 6 hours at day 2 of adulthood to wild type animals treated with Safingol (130 µM), they normally turned pale when deprived of food but were incapable of regaining their fat as fast as the non treated control (Figure 5C). Thus, Safingol treatment on wild type animals at 130µM can recapitulate the fat phenotype triggered by RNAi against *sphk-1.* The inventors conclude that Safingol can recapitulate the inhibition of *sphk-1* by RNAi (Figure 5C).

In summary the inventors showed that DR is reversible and identified SPHK-1 as key determinant of this reversibility. Consequently, they show that lowering the activity of *sphk-1* specifically promote the transcriptional response to DR. This allows benefiting the positive effects of DR by performing a short DR only. This treatment delays aging, increases leanness associated to DR and diminishes tumor formation. Finally, the inventors show that chemical inhibition of *sphk-1* can have similar effects. Further characterization of these findings on mammalian system is underway. The inventors therefore propose a new pharmacological strategy based on the coupling of a slight inhibition of the *sphk* enzyme with a short DR.

### Material and methods

**Worm strains and strain maintenance :** *C. elegans* strains were maintained on standard nematode growth medium (NGM) plates containing OP50 bacterial lawns as food source. The strains used in this study are described in the following table:

| Strain name | Origin | Strain number (allele or trangene) |
|---|---|---|
| N2 | | Wild type |
| Daf-16:GFP | CGC* | TJ356 (zls356) |
| Skn-1:GFP | gift from Keith Blackwell | LD 001 (Is007) |
| Myo3:GFP | CGC | SJ4103 (zcls14) |
| SPHK1 mutant | CGC (backcrossed once) | VC916 (ok1097) |
| | Backcrossed 3 times more in the inventors' lab | |
| Q35:YFP | gift from Francesca Palladino | AM140 (rmls132) |

| | | |
|---|---|---|
| * CGC = Caenorhabditis Genetics Center | | |

**Solutions -** *Nematode Growth Medium (NGM):* Agar 20g/L, NaCl 3g/L, bactopeptone 2,5g/L, cholesterol 5mg/L, Phosphate buffer pH 6 (KH₂PO₄/K₂HPO₄) 25 mM, MgSO₄ 1mM, CaCl₂ 1mM. *M9 buffer:* KH₂PO₄ 3g/L, Na₂HPO₄ 6g/L, NaCl 5g/L, MgSO₄ 1 mM. *Bleaching solution* (for a 50ml solution): 10ml sodium hypochlorite 2.6%, 15 ml water, 25ml NaOH 1M. *S-basal:* NaCl 5.8g/L, Phosphate buffer pH 6 (KH₂PO₄/K₂HPO₄) 50 mM, cholesterol 5mg/L.
**RNA interference by feeding -** RNAi-expressing bacteria were obtained from the Ahringer library. Bacterial clones were grown overnight at 37°C in LB containing Ampicillin (50 µg/ml) and Tetracyclin (12.5 µg/ml) and seeded onto NGM plates containing carbenicillin (25 µg/ml). RNAi expression was induced by adding IPTG (1mM) on top of already seeded plates. L1 worms were placed on these plates and incubated at 20°C until adulthood. The sequence encoding RNAi against *sphk-1* is as disclosed in SEQ ID No: 15.
Synchronization of worms (Bleaching) - Adult worms were resuspended in M9 buffer. After centrifugation at maximum speed for 2 min and removal of the supernatant worms were incubated in 10 ml bleaching solution and vortexed until dissolution (about 6-7 min). Eggs were washed 4 times with M9 buffer by centrifugation at maximum speed for 2 minutes at +4° C between each wash. Eggs were then transferred to 250 ml flask in 40 ml M9 buffer and incubated overnight at 20°C under agitation. Synchronized population of L1 worms were transferred to plates seeded with bacteria.
**Lifespan analysis -** Lifespan studies were performed according to standard protocols (Kenyon et al., 1993). Worm lifespan assays were performed at 20°C. A synchronized population of L1 worms was transferred to NGM plates containing Carbenicillin (25 µg/ml) and 5-fluorouracil at 15µM (FU prevents the development of progeny) already seeded with HT115 or RNAi-expressing bacteria. Induction of RNAi expression with IPTG was performed a few hours before transferring the worms on plates. Approximately 10-20 hermaphrodites were cultured on each Petri dish. For each lifespan assay, about 100 worms were analyzed from day 1 of adulthood until death. Worms were changed to new plates at day 8. The number of alive, dead, missing, exploded and bagged animals was scored every other days. Raw data were analyzed using the Biopylife software, especially developed for the inventors' lab by students of the Institut National des Sciences Appliqués of Lyon in 2008.
**Mitochondria staining -** The diOC₆ vital dye was used to stain mitochondria in worms. This dye was used at low concentration as it can also stain the endoplasmic reticulum at higher concentrations. Plates were prepared by adding the diOC₆ solution at 2 µg/ml on top of plates already seeded with bacteria. diOC₆ was diluted in water from a stock solution at 2 mg/ml in DMSO and kept at -80°C. Adult worms were transferred on these plates seeded with either HT115 (empty vector) or RNAi-expressing bacteria. Worms were incubated overnight at 20°C and observed the next day using a confocal microscope. There was no washing step required before microscopy. To visualize mitochondria, the inventors also used Myo3:GFP transgenic worms that expressed a robust GFP signal in muscle mitochondria. Worms were synchronized as described before. L1 worms were placed on plates seeded with HT115 or RNAi-expressing bacteria and incubated at 20°C until adulthood.
**Microscopy -** Living worms were immobilized on slides coated with poly-lysine and in a solution of 0.2mM Levamisol to paralyze them prior to observation under the microscope. Pictures were taken using an axioplan Zeiss CDD or a confocal spectral Leica microscope (PLATIM facilities available at the ENS).
**DAF-16 localization -** A DAF-16:GFP strain was used to visualize the localization (nuclear or cytoplasmic) of the daf-16 protein in worms and to compare its localization when worms were grown on HT115 (empty vector) or on RNAi against SPHK-1. Synchronized L1 larvae were transferred on NGM plates seeded with HT115 or RNAi against SPHK1 and worms were incubated at 20°C until day 1 of adulthood (d1). At d1 half of the worms are transferred to NGM plates (no seeded bacteria) for 6 hours to put the worms in DR conditions and half of the worms are kept on the same NGM plates seeded with bacteria (AL condition). After 6 hours about 20 worms per condition were mounted on slides and observed under the microscope immediately after mounting (as daf-16 is known to be very responsive to stress, which causes its translocation to the nucleus). DR worms were then transferred back on NGM plates seeded with HT115 or RNAi against SPHK1 and incubated for 15 hours at 20°C prior to the next observation under the microscope.
**Quantitative RT-PCR -** *RNA extraction:* Total RNA was isolated from synchronized populations of day-1 reproductive adults (about 3000 adult worms per condition) using the following method: worms were harvested with M9 buffer and washed 3 times with M9 buffer to remove bacteria. Worms were washed twice more with DEPC water. The final worm pellet was made as small as possible (usually 100 µl). 200 µl of TRI reagent (MRC) was added to the worms (the ratio Trizol/worms was approximately 2/1) and the mixture was vortexed for at least 1 min to resuspend the worms. The mixture was frozen at -80°C overnight or for a longer period prior to the next RNA extraction steps. Frozen worms were then placed on ice and vortexed for 5 min to freeze crack worms. Tubes were let at room temperature to allow the worms to thaw and settle. The trizol/worm mix was transferred to eppendorf tubes and 40 µl of Chloroform (200µl per 1 ml trizol/worm mix) was added to each tube. After shaking the tubes vigorously by hand for 15 sec and letting them incubate at room temperature for 2 to 3 min, the tubes were centrifuged at 12000 g for 5min. The upper aqueous phase was carefully transferred to a new tube and its volume was estimated (usually about 3 times the volume of chloroform used). An equal volume of 70% ethanol (prepared in DEPC water) was slowly added to the aqueous phase and the tubes were mixed gently by inversion. The mixture was transferred to mini Rneasy binding columns (Qiagen). From this step the RNA extraction was performed by following the Rneasy kit instructions, including the extra step of DNAse I digestion on column. Final RNAs were eluted with 30µl RNAse free water. RNA concentrations were measured using a nanodrop spectrophotometer. *Reverse Transcription :* cDNA was created using the RevertAid First Strand cDNA synthesis kit (Fermentas reference K1622, see attached documents RT1 and RT2). The procedure described in the manual was followed. Briefly, each reaction was performed on 500ng of RNA. A PCR machine was used to incubate the samples at different temperatures. For each reaction, 500ng of diluted RNA was incubated with 1µl of random primer (final volume 12µl with water). Tubes were incubated at 70°C for 5min. After the addition of 4µl 5X reaction buffer, 1µl Ribonuclase inhibitor and 2µl dNTPs, tubes were incubated at 25°C for 5min. After the addition of 1µl enzyme, tubes were finally incubated at 25°C for 10 min, then 42°C for 60 min and 70°C for 10 min. The final cDNAs were kept at -20°C. *Q-PCR Reaction:* SybrGreen real-time Q-PCR experiments were performed as described in the LightCycler FastStart DNA Master^{Plus} SYBR Green I manual using a 1.5 Light Cycler (Roche) The protocol was slightly modified for the PCR mix (see table below and attached document QPCR). All Q-PCR experiments were normalized to act-1 mRNA levels. See attached document QPCR program for details of the Light cycler program.

| | | |
|---|---|---|
| Gene | QPCR primers | Hybridization temperature (°C) |
| SPHK1 | L- acaggaacaatgtcgtggaaa (SEQ ID No 1) | 60 |
| | R- tttatccaacttcggtccaac (SEQ ID No 2) | |
| C34C6.4 | L- cggcacgatctctacaaagg (SEQ ID No 3) | 60 |
| | R- agcacttccagctccaacaa (SEQ ID No 4) | |
| DAF16 | L- tacgaatggatggtccagaa (SEQ ID No 5) | 58 |
| | R- tcgcatgaaacgagaatgaa (SEQ ID No 6) | |
| PHA4 | L- aacgtgagccatcgagaaag (SEQ ID No 7) | 60 |
| | R- gatgatgtggtcgtgatgga (SEQ ID No 8) | |
| AAK2 | L- agctcgtcgcttctttcaac (SEQ ID No 9) | 58 |
| | R- caaagtccgcaatcttcaca (SEQ ID No 10) | |
| LET 363 | L- cgccctgattctcaatgaac (SEQ ID No 11) | 60 |
| | R- ggacaagccattcaacacct (SEQ ID No 12) | |
| ACT1 | L- cccactcaatccaaaggcta (SEQ ID No 13) | 58 or 60 |
| | R- atctccagagtcgaggacga (SEQ ID No 14) | |

| Components | Volume for a 10µl reaction |
|---|---|
| Water, PCR grade | 6 µl |
| PCR primers 10X | 1µl |
| Master Mix 5X | 2 µl |
| Total Volume | 9 µl |

Each pre-cooled capillary was filled with 9 µl of PCR mix and 1 µl of cDNA template at a 1:30 dilution (or 1:10; 1:90, 1:270 dilutions for the standards).

**SKN-1:GFP** - The strain Is007 (SKN-1:GFP) was used to visualize the activation/deactivation of skn-1 in the ASI neurons depending on the feeding conditions. Worms were grown as previously described on HT115 or RNAi. Fluorescent images were collected from worms at various days of life, fed AL or in DR conditions. The quantification of the mean fluorescent signal in the ASI neurons was performed using the NIH Image J software.

**GLD-1 DAPI -** DAPI stainings were performed on worms grown on HT115 or *gld-1* RNAi from L1 (synchronized population on N2 worms) to adulthood. *Staining of whole animals :* worms were first washed in M9 buffer to remove bacteria. The DAPI staining of whole animals was directly performed in 15ml conical tubes. Animals were fixed in 3 ml cold Methanol (kept at -20°C) for 5 min (tubes were placed for 3 min at -20°C and then centrifuge at +4°C for 2 min). It's important not to leave the worms for too long in Methanol as it can destroy all tissues. Worms were washed 3 times in PBT (PBS containing 0.1% tween 20) and then incubated in 1 ml 100ng/ ml DAPI solution (prepared by mixing 1 µl of stock solution kept at +4°C and 2ml of PBT) for 20 min at room temperature. After 3 washes in PBT, worms were mounted on agarose pads or poly-lysine coated slides. Slides were sealed and kept at +4°C until observation. *Staining of isolated gonads:* For DAPI staining of isolated gonads, worms were first incubated in ½ M9 buffer (50% water, 50% M9) containing 0.2 mM Levamisol for a few minutes. Drops of liquid containing paralyzed animals were put on poly-lysine coated slides. Head of animals were cut near the pharynx region using a needle attached to a syringe, which allow the gonads to easily extrude from the bodies of worms. The DAPI staining of isolated gonads was directly performed on slides. Isolated gonads were fixed in PFA 3% (prepared from a ParaFormAldehyde 16% stock solution diluted to 3% in 0.1M K₂HPO₄ pH 7.2 and kept at -20°C) for 10 min. Gonads were carefully washed 3 times in PBT. Gonads were stained with 100ng/ ml DAPI solution for 5 min and finally washed 3 times in PBT. A coverslip was directly added on top of the slide. Slides were sealed and kept at +4°C until observation.

**Screen that allowed the inventors to identify SPHK1 as a genetic determinant for fat re-accumulation after SDR -** *General protocol:* Day 1: RNAi clones were taken out of the -80°C library. Bacteria were incubated overnight at 37°C in LB containing Ampicillin (50 µg/ml) and Tetracyclin (12.5 µg/ml). Day 2 : 150 µl of each RNAi clones were spread on NGM plates containing Carbenicillin (25 µg/ml). Plates were left at room temperature until dried. Adult N2 worms were bleached and eggs were maintained overnight in M9 buffer at 20°C under agitation. Day 3 - RNAi expression was induced by adding 150 µl IPTG (1mM) on top of already seeded and dried plates a few hours before putting L1 worms on these plates. M9 buffer containing L1 larvae was centrifuged at maximum speed for 5 min. After removal of the supernatant the number of L1 was estimated. About 100 to 150 L1 worms were put on each dry plate seeded previously with RNAi bacteria and induced with IPTG. Plates were incubated at 20°C until worms became adults and started laying eggs. Day 6 - Worms were observed to see if they all develop into adults and to detect any abnormality (larval arrest, clear phenotype...). Worms were removed from each plate with M9 buffer and transferred to 15ml conic tubes using a 5 ml pipette. Once worms settled down naturally to the bottom of the tube, the supernatant was removed using a vacuum pump and worms were kept in a small volume of M9 buffer (100 µl). Worms were then transferred to empty NGM plates containing kanamycin (25µg/ml) for 6 hours at 20°C. After 6 hours of starvation, worms were observed to check if they all looked clear (disappearance of fat). They were then resuspended in M9 buffer, transferred to 15 ml conical tubes, left to sediment to the bottom of the tube, kept in a small volume of M9 buffer and finally transferred to new NGM plates already seeded with RNAi and induced by IPTG a few hours before. Worms were incubated about 15 hours at 20°C. Day 7 - Worms were carefully observed and clones that still exhibited a clear phenotype after 15 hours re-feeding (less fat re-accumulated compared to worms on HT115) were selected for further analysis.

### Example 2 : Method of screening.

If not specified otherwise all reagents and agar dishes should be left at room temperature (between 20 to 25°c). *Worms's preparation :* 100 starved N2 worms were disposed onto fresh NGM plates (∅ 10cm) containing OP50 bacterial strain. When worms are adult, they were bleached to obtain L1 larvae as follow: (1) They were removed from the plate with 4ml of M9 buffer, (2) disposed onto a 14ml falcon qsp 14ml with M9 buffer, (3) washed 3 times to discard all bacteria by repetitive centrifugation and M9 addition. Finally, worms were centrifuged 2min at 4300 rpm to collect all worms plus their eggs (break ≤ 2, to avoid risk of eggs resuspension because of a sharp deceleration). 13ml of supernatant is then discarded and 10ml of bleach solution is added (10ml Bleach, 15ml H2O, 25ml NaOH 1N). After heavy vortexing (until worms explode; no more than 6 min, more than 6 min will altered eggs structure and destroy them), the mix is centrifuged 2min 4300rmp at 4°c (4°c reducing bleach solution activity). The supernatant is discarded and remaining eggs are washed 4 times with at least 14ml of cold M9 buffer. Finally eggs are resuspended in 50ml M9 buffer in a 250ml Erlen where they are left overnight to incubate at 20°c. This allows hatching of L1 larvae. Larvae arrest growth due to nutrient limitation. About 300 L1 larvae are disposed onto fresh NGM plates (∅ 20cm) seeded with OP50 (One 96-wells plate takes 7 ∅ 20cm plates). Plates containing worms are then incubated at 20°c until day 1 of adulthood (3 days). Worms are then ready to go onto NGM plates containing no bacteria for 6 hours (shorter starvation does not allow a full loss of fat and will impair analysis. A longer starvation will lead to development of eggs within the adult altering the analysis). The switch to empty plates is performed as followed: Plates are washed with M9 buffer four times in 50 ml falcon tubes and finally resuspended in M9 to reach a final concentration of 20 worms per 10µl. *Switch:* inoculate a 96-wells plate with 190µl of M9 buffer containing drugs (final concentration 130µM) and add 10µl of worms (20 worms allows a easy detection of the fat re-accumulation phenotype) and incubate at 20°c 150 rpm for 6 hours. After 6 hours of food deprivation, put 96-wells plates on bench for 3min until all worms settle at the bottom of the wells and discard 190µl of M9 (be careful to always pipette on the top of the liquid, in order not to aspire worms) and add 190µl of axenic medium containing drugs in each well. After incubation at 20°c overnight at 150rpm (The incubation time is needed to let fat re-accumulate, worms can be scored as follows: after waiting for worms to sediment at the bottom of the wells, discard 190µl (be careful to always pipette on the top of the liquid, in order not to aspire worms) of axenic medium. Low liquid levels allow worms to move less and facilitate their observation. Worms are observed under a microscope and fat content is scored by simply assessing how clear/dark worms are. The darker the fatter.

**Medium composition -** *LB:* 10g Tryptone; 5g Yeast extract; 10g de NaCl; Qsp 1L H2O; autoclave. *Nematode Growth Medium:* 3g de NaCl; 2,5g de bactopeptone; 975ml H2O; autoclave. Then add 1ml of cholesterol 5mg/ml and 1ml of CaCl2 1M. Stir for 1min and add 1ml of MgSo4 1M. Stir for 1min and add 25ml of phosphate buffer 1M. *M9*: 3g KH2PO4; 6g Na2HPO4; 5g NaCl; qsp 1L H2O; stir for 5min and add 1ml MgSO4 1M; autoclave. *Phosphate buffer 1M:* 108,3g KH2PO4; 35,6g K2HPO4; qsp 11 H2O. Adjust pH to 6 with KOH. Autoclave. *Axenic medium:* 3% (w/v) soy peptones; 3% (w/v) yeast extract; Autoclave. Add 1X hemoglobin (with 100X stock; 100X = 5g Hemoglobin in 100ml KOH 0,1N) and filter at 0,22 micron.

**Table 1**

| Gene | Suppression of prolonged leanness phenotype | Suppression of prolonged lifespan phenotype |
|---|---|---|
| PHA-4 : forkhead transcription factor that mediates the DR response | Yes | Yes |
| DAF-16 : forkhead transcription factor that mediates the IIS pathway | Yes | No |
| SKN-1 : forkhead transcription factor required for the DR response | Yes | ? |
| CYC-1 : genes involved in mitochondrial mediated longevity | No | No |
| LET-363 (TOR) : kinase that extends longevity when knocked down and potential candidate for DR | No | No |
| AAK-2 (AMPK) : genes that extends longevity when knocked down and potential candidate for DR | No | No |
| LET-60 (RAS) : genes that extends longevity when knocked down and potential candidate for DR | No | No |

### REFERENCE

An, J.H., and Blackwell, T.K. (2003). Genes Dev 17, 1882-1893.
An, J.H., et al. (2005). Proc Natl Acad Sci U S A 102, 16275-16280.
Antebi, A. (2007). Nature 447, 536-537.
Baur, J.A., and Sinclair, D.A. (2006). Nat Rev Drug Discov 5, 493-506.
Bishop, N.A., and Guarente, L. (2007). Nature 447, 545-549.
Bowerman, B., et al. (1992). Cell 68, 1061-1075.
Buehrer, B.M., and Bell, R.M. (1992). J Biol Chem 267, 3154-3159.
Colman, R.J., et al. (2008). Biol Sci Med Sci 63, 556-559.
Delgado, A., et al. (2006). Biochim Biophys Acta 1758, 1957-1977.
Dirks, A.J., and Leeuwenburgh, C. (2006). Mech Ageing Dev 127, 1-7.
Fontana, L., et al. (2004). Proc Natl Acad Sci U S A 101, 6659-6663.
Friedman, J.R., and Kaestner, K.H. (2006). Cell Mol Life Sci 63, 2317-2328.
Greer, E.L., et al. (2007). Curr Biol 17, 1646-1656.
Hait, N.C., et al. (2006). Biochim Biophys Acta 1758, 2016-2026.
Kenyon, C., et al. (1993). Nature 366, 461-464.
Kim, J.W., et al. (2005). Bioorg Med Chem 13, 3475-3485.
Kirkwood, T.B. (2005). Cell 120, 437-447.
Lamont, L.B., et al. (2004). Dev Cell 7, 697-707.
Li, J., et alA. (2007). Methods Mol Biol 399, 67-78.
Mair, W., and Dillin, A. (2008). Aging and Survival: The Genetics of Life Span Extension by Dietary Restriction. Annu Rev Biochem.
Mair, W., et al. (2003). Science 301, 1731-1733.
Mango, S.E., et al. (1994). Development 120, 3019-3031.
Masoro, E.J. (2002). Caloric restriction : a key to understanding and modulating aging, 1st edn (Amsterdam ; Boston, Elsevier).
McCay, C.M., et al. (1935). J Nutr 10, 63-79.
Mizugishi, K., et al. (2005). Mol Cell Biol 25, 11113-11121.
Panowski, S.H., et al. (2007). Nature 447, 550-555.
Pearson, K.J., et al. (2008). Proc Natl Acad Sci U S A 105, 2325-2330.
Perez, C.L., and Van Gilst, M.R. (2008). Cell Metab 8, 266-274.
Pinkston, J.M., et al. (2006). Science 313, 971-975.
Smith, E.D., et al. (2008). BMC Dev Biol 8, 49.
Spiegel, S., and Milstien, S. (2007). J Biol Chem 282, 2125-2129.
Sutphin, G.L., and Kaeberlein, M. (2008). Exp Gerontol 43, 130-135.
Walford, R.L., et al. (1999). Toxicol Sci 52, 61-65.
Walker, A.K., et al. (2000). J Biol Chem 275, 22166-22171.
Weyer, C., et al. (2000). Am J Clin Nutr 72, 946-953.

## Claims

1. A sphingosine kinase inhibitor for prolonging the benefits of a Dietary Restriction in a subj ect.

2. The sphingosine kinase inhibitor of claim 1, wherein the sphingosine kinase inhibitor is to be administered during the period of Dietary Restriction and after the end of this period.

3. The sphingosine kinase inhibitor of claim 1 or 2, wherein the benefits are selected from the group consisting of a decrease of the incidence of aging related pathologies and an increased vitality and leanness.

4. The sphingosine kinase inhibitor of claim 1, wherein the benefits are a decrease of body mass.

5. The sphingosine kinase inhibitor of any one of claims 1-4, wherein the subject is an adult, preferably a middle aged subject or an elderly.

6. The sphingosine kinase inhibitor of any one of claims 1-5, wherein the sphingosine kinase inhibitor is selected from the group consisting of a siRNA specific of the sphingosine kinase, a sphingosine kinase dominant negative and a chemical compound.

7. A therapeutic or non-therapeutic method for prolonging the benefits of a Dietary Restriction in a subject, comprising submitting the subject to a Dietary Restriction and administering to the subject an effective amount of an inhibitor of a sphingosine kinase during the Dietary Restriction and after the end of the Dietary Restriction.

8. The method of claim 7, wherein the benefits are selected from the group consisting of a decrease of the incidence of aging related pathologies, an increased vitality and leanness and a decrease of the body mass.

9. The method of claim 7 or 8, wherein the subject is an adult, preferably a middle aged subject or an elderly.

10. A product comprising a sphingosine kinase inhibitor and at least one component selected from the group consisting of beverage ingredients, food ingredients, animal feed ingredients, pet food ingredients, nutraceutical ingredients, dietary supplement ingredients, and functional food ingredients.

11. A method for identifying or screening a molecule suitable for prolonging the benefits of a Dietary Restriction, comprising:
1) providing adult worms with a normal fat content;
2) submitting the worms to food deprivation during about 4-7 hours in the presence of a test molecule;
3) cultivating the worms in an axenic medium containing the test molecule during about 15-25 hours;
4) determining the fat content of worms; and,
5) selecting the test compound if the fat content of worms is low, said selected compound being suitable for prolonging the benefits of a Dietary Restriction.

12. The method according to claim 11 wherein a worm with a normal fat content is dark and a worm with a low fat content is clear, and the determining step 4) is based on this parameter.

13. The method according to claim 11 wherein the determining step 4) is performed automatically thanks to a large particle FACS machine and the discriminating parameter is optical density and/or autofluorescence.

14. The method according to any one of claims 11-13, wherein the method is carried out in a multiple-well plate.

15. The method according to any one of claims 11-14, wherein the worms are *C*. *elegans.*
